# EUROPÄISCHE PATENTSCHRIFT

(11) **EP 0 633 250 B1**
(45) Veröffentlichungstag und Bekanntmachung des Hinweises auf die Patenterteilung: **12.02.1997**
(21) Anmeldenummer: 94106176.4
(22) Anmeldetag: 21.04.1994
(51) Int. Cl.: C07D 213/79, C07D 213/84

(54) **Verfahren zur Herstellung von 2,4-Pyridindicarbonsäure**
Process for the preparation of 2,4-pyridinedicarobxylic acid
Procédé pour la préparation d'acide pyridinedicarboxylique-2,4

(30) Priorität: 10.07.1993 DE 4323071
(43) Veröffentlichungstag der Anmeldung: 11.01.1995
(73) Patentinhaber: Weyl GmbH, 68305 Mannheim (DE)
(72) Erfinder: Sendelbach, Stefan, Dr., D-74172 Neckarsulm (DE); Orth, Winfried, Dr., D-67454 Hassloch (DE); Weiss, Wolfgang, Dr., D-68535 Edingen-Neckarhausen (DE); Kleffner, Hans Werner, Dr., D-67271 Battenberg (DE)
(74) Vertreter: Patentanwälte Zellentin & Partner

(56) Entgegenhaltungen:
- CHEMICAL ABSTRACTS, vol. 55, no. 7, 1961, Columbus, Ohio, US; abstract no. 6478b, H. TANI 'Reaction of N-alkoxypyridinium derivatives.'
- JOURNAL OF THE CHEMICAL SOCIETY, CHEMICAL COMMUNICATIONS., Nr.5, 1990, LETCHWORTH GB Seiten 370 - 371 C.W. SOMAWARDHANA ET AL. 'Novel solid-state support for radio synthesis of radiopharmaceuticals labelled with [11C]-cyanide.'
- BULLETIN OF THE CHEMICAL SOCIETY OF JAPAN, Bd.43, Nr.10, 1970, TOKYO JP Seiten 3210 - 3214 E. MATSUMURA ET AL. 'Studies of the Reissert-Kaufmann type reaction of 4-nitropyridine N-oxyde and its homologues.'
- R.A. ABRAMOVITCH 'Chemistry of heterocyclic compounds. Vol. 14: Pyridine and its derivatives. Supplement part 3.' 1974 , WILEY , NEW YORK * Seite 300 *
- CHEMICAL AND PHARMACEUTICAL BULLETIN, Bd.7, 1959, TOKYO JP Seiten 930 - 934 H. TANI 'The reaction of N-alkoxypyridinium derivatives.'

## Beschreibung

Die Erfindung betrifft ein Verfahren zur Herstellung von 2-Cyanoisonicotinsäure und 2,4-Pyridindicarbonsäure aus Isonicotinsäure, wobei als Zwischenprodukt Isonicotinsäure-N-oxid hergestellt wird.

Beide Verbindungen stellen wertvolle Zwischenprodukte für die Synthese pharmazeutischer Wirkstoffe dar. Beispielsweise wird 2,4-Pyridindicarbonsäure zur Herstellung von Arzneimitteln zur Hemmung der Prolin- und Lysinhydroxylase oder als Zwischenprodukt für Fibrosuppressiva und Immunsuppressiva verwendet. 2-Cyanoisonicotinsäure wiederum läßt sich als Zwischenprodukt zur Herstellung von 2,4-Pyridindicarbonsäure verwenden.

Aus der Literatur sind verschiedene Methoden zur Herstellung von 2,4-Pyridindicarbonsäure bekannt. Beispielsweise erfolgt die Bildung der Dicarbonsäure aus 2,4-Dimethylpyridin beim Erhitzen mit SeO₂ in 3-Methylpyridin bei 110 °C oder in Essigsäureethylester, Methanol oder Xylol unter Rückfluß, wobei eine Ausbeute von 67 % erzielt werden kann (Jerchel et al.; Liebigs Annalen der Chemie, 613 (1958), 153, 155, 162; Marcot, B.; Palland, R.; Compt. rend. 248 (1959), 252 - 4).

Auch beim Erhitzen von 2,4-Dimethylpyridin in Gegenwart von Selen und konzentrierter Schwefelsäure wird die Dicarbonsäure gebildet, jedoch nur in einer theoretischen Ausbeute von 29 % (Ochiai, E.; Okuda, S.; J. Pharm. Soc. Japan 70 1950, 156 - 61).

Nach heutigen Reinheitsanforderungen, die an Zwischenprodukte zur Herstellung von pharmazeutischen Wirkstoffen gestellt werden, sind diese Methoden jedoch nicht durchführbar, da auch nach aufwendigen Reinigungsmaßnahmen immer noch Spuren des verwendeten toxischen Selens oder Selendioxids im Produkt verbleiben.

Zwar kann durch Oxidation von 2,4-Dimethylpyridin mit konzentrierter Salpetersaure bei 185 bis 190 °C unter Druck 2,4-Pyridindicarbonsäure in einer Ausbeute von 65 % erzielt werden, jedoch ist diese Methode nicht in einfacher Weise im technischen Maßstab durchführbar, da die Oxidation aufgrund der starken Reaktivität in 2,4-Position alkylierter Pyridine nur unter kontrollierten Bedingungen durchgeführt werden kann, und die Bildung nitroser Gase besondere Sicherheitsmaßnahmen erfordert.

Als weitere Methode wird in der Patentschrift DBP 10 10 524 eine Oxidation mit Sauerstoff in Gegenwart äquimolarer Mengen Kupfernitrat in wäßriger Lösung bei 225 bis 230 °C und 60 at beschrieben. Die durch die Oxidation gebildete Dicarbonsäure fällt dabei als Kupfersalz aus. Die Freisetzung der Säure erfolgt über die Bildung des Natriumsalzes, das anschließend mit Salzsäure zersetzt wird. Zwar fällt bei dieser Methode das Kupfersalz erst in verhältnismäßig hoher Ausbeute an, durch die zweistufige Freisetzung der Säure geht jedoch wiederum Produkt verloren, und, was für die weitere Verwendung für pharmazeutische Produkte von Bedeutung ist, es bleiben trotz mehrmaligen Umkristallisierens Spuren von Kupfer im Produkt erhalten, so daß diese Methode für diesen Zweck als nicht geeignet erscheint.

Auch die Freisetzung der Säure mit Hilfe von Schwefelwasserstoff unter Bildung schwerlöslichen Kupfersulfids, wie es verschiedentlich beschrieben worden ist, eignet sich nicht, da dem erhaltenen Produkt einerseits noch H₂S anhaftet, andererseits das Arbeiten mit H₂S aufgrund seiner Toxizität besondere Sicherheitsvorkehrungen bedarf.

Die Oxidation von 2,4-Dimethylpyridin mit Kaliumpermanganat führt zur Bildung von großen Mengen schwer entsorgbaren Braunsteins und ist wegen geringer Ausbeuten unwirtschaftlich (H. Meyer, H. Tropsch, Monatshefte Chemie 35 (1915), 189).

Es besteht daher die Aufgabe, ein neues, wirtschaftliches Verfahren zur Verfügung zu stellen, wodurch sich 2,4-Pyridincarbonsäure in hoher Ausbeute und Reinheit darstellen läßt, so daß es ohne aufwendige Reinigungsverfahren als Zwischenprodukt zur Herstellung pharmazeutischer Produkte verwendet werden kann.

Die Lösung der Aufgabe erfolgt durch ein Verfahren gemaß Anspruch 1 und seiner besonderen Ausgestaltung gemäß der Ansprüche 2 bis 6, das Zwischenprodukt 2-Cyanoisonicotinsäure gemäß Anspruch 7 und seine Verwendung zur Herstellung von 2,4-Pyridindicarbonsäure gemäß Anspruch 8.

Da bislang durch bekannte Oxidationsmethoden 2,4-Pyridindicarbonsäure entweder nur in niedrigen Ausbeuten oder auch nach hohem Reinigungsaufwand nur in nicht ausreichender Reinheit, bzw. nur unter aufwendigen Sicherheitsvorkehrungen zu erhalten war, wurde nach einem alternativen, einfach durchzuführenden preiswerten Syntheseweg gesucht, wodurch 2,4-Pyridindicarbonsäure in hoher Ausbeute und hoher Reinheit hergestellt werden kann, so daß es als Zwischenprodukt für die Herstellung pharmazeutischer Wirkstoffe verwendet werden kann.

Aufgrund der zurückgehenden Stahlproduktion ist auf dem Markt eine Verknappung des indirekt dabei anfallenden Teers und somit auch für die daraus bisher durch Destillation gewonnenen Alkylpyridine, insbesondere für das 2,4-Dimethylpyridin eingetreten. Daher bieten sich als Ausgangsverbindungen zur Synthese von 2,4-Pyridindicarbonsäure preiswerte, durch Cyclisierung und evtl. anschließende Reaktionen erhaltene käufliche Pyridinderivate, wie 4-Pyridincarbonsäure, auch Isonicotinsäure genannt, 4-Pyridincarbonitril oder 4-Pyridincarboxamid, Isonicotinsäureamid genannt, an.

Die Bildung der 2,4-Pyridindicarbonsäure kann theoretisch aus diesen Verbindungen nach folgendem Schema erfolgen, vorausgesetzt, die bisher nicht bekannten Derivate 2-Cyanoisonicotinsäure und 2-Cyanoisonicotinsäure lassen sich herstellen.

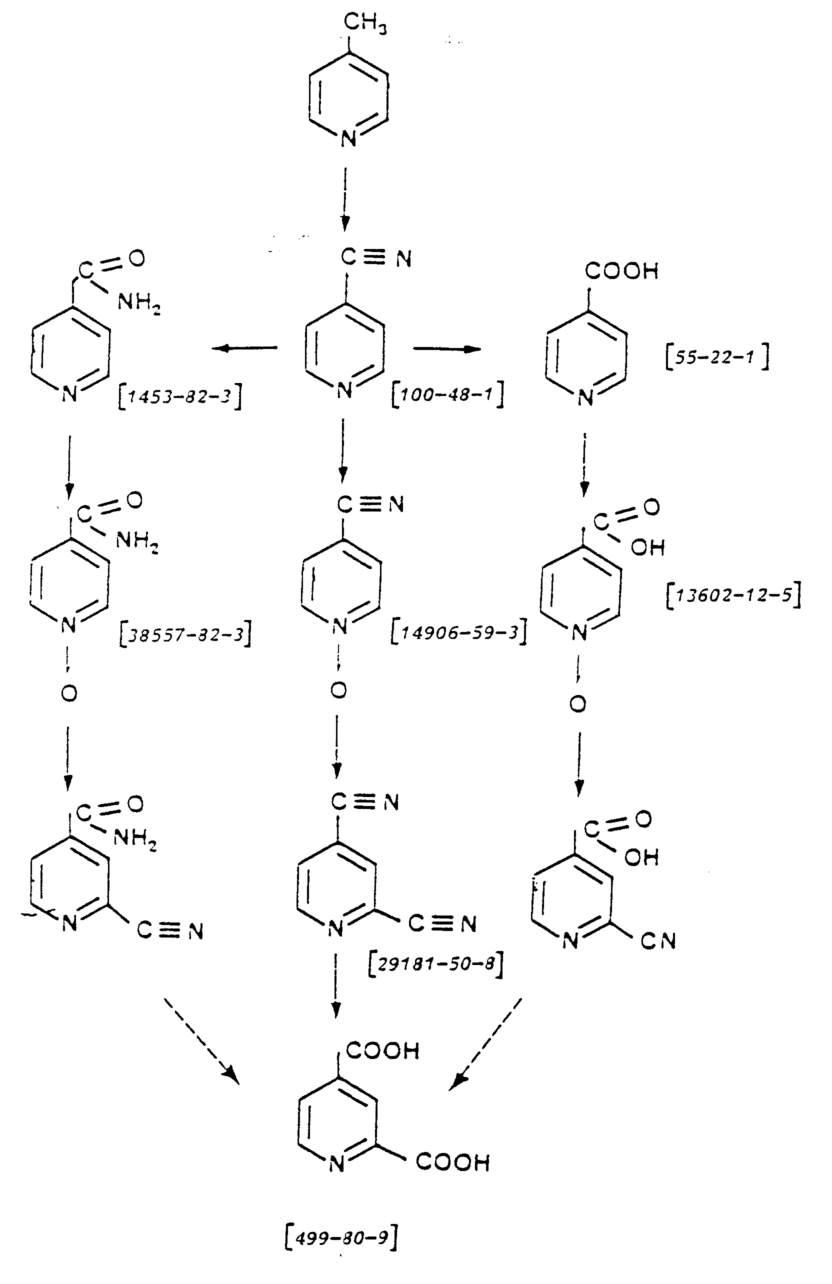

Aus Chemical Abstracts 55: 6478b ist bekannt, Isonicotinsäuremethylester-N-oxid in 2-Stellung zu cyanisieren, den resultierenden 2-Cyanoisonicotinsäuremethylester mit Alkali zu behandeln und das Reaktionsgemisch anschließend zu verestern. Der Fachmann könnte versuchen, bei diesem Reaktionsweg die Veresterungsstufe einzusparen und statt dessen die im Reaktionsgemisch erwartete 2,4-Pyridindicarbonsäure aufzuarbeiten. Jedoch läßt dies keine allzu hohe Ausbeute erwarten, da bereits die erste Teilreaktion, die Cyanisierung, trotz molarem Überschuß an Cyanid nur eine Ausbeute des Zwischenproduktes von 69 % ergibt.

Überraschenderweise zeigte sich nun, daß sich 2,4-Pyridindicarbonsäure sowohl aus dem kommerziell verfügbaren Isonicotinamid als auch aus der preiswerten Isonicotinsäure in hohen Ausbeuten und hoher Reinheit gewinnen läßt, ohne die gebildeten Zwischenprodukte aufwendig reinigen zu müssen.

Versucht man dagegen die Dicarbonsäure aus dem 4-Pyridincarbonitril herzustellen, ist bereits die Synthese des Zwischenproduktes Pyridin-2,4-dicarbonitril mit der Bildung eines hohen Anteils teerartiger Nebenprodukten verbunden, so daß aufwendige Reinigungsstufen zwischengeschaltet werden müssen und die Ausbeute letztendlich für die Durchführung im technischen Maßstab zu niedrig ist.

Da die Darstellung aus Isonicotinamid Gegenstand einer separaten Anmeldung ist, soll dieser Weg hier nicht berücksichtigt werden.

Zur Herstellung der 2,4-Pyridindicarbonsäure aus Isonicotinsäure kann der aromatische Ring am Stickstoffatom nach bekannter, in der Literatur beschriebener Methode oxidiert werden. In hohen Ausbeuten kann dies z. B. in Gegenwart von Wasserstoffperoxyd in Essigsäure (analog Liberman
et al., Bull. Soc. Chim. France 1958, Seiten 694 und 698), oder in Peressigsäure erfolgen. Das dabei anfallende kristalline Isonicotinsäure-N-oxid wird ohne weitere Reinigung nach einer in US 2 991 285 beschriebenen Methode mit einem Alkylierungsmittel zum N-Alkoxy-isonicotinsäuresalz umgesetzt. Als Alkylierungsmittel sind die gängigen Alkylierungsmittel einsetzbar, insbesondere Dimethyl- und Diethylsulfat.

Die Umsetzung kann in einem inerten Lösungsmittel, wie z. B. Toluol, aber auch ohne Zusatz eines Lösungsmittels bei einer Temperatur im Bereich von 90 bis 105 °C erfolgen.

Nach beendeter Reaktion kann das gebildete Pyridiniumsalz, welches durch spektroskopische Methoden, wie z. B. NMR, eindeutig charakterisiert werden kann, direkt zur 2-Cyanoisonicotinsäure umgesetzt werden. Zu diesem Zweck wird das Salz in Wasser aufgenommen und mit einer wäßrigen Alkalicyanidlösung, bevorzugt einer Natrium- oder Kaliumcyanidlösung, vermischt. Die Umsetzung verläuft problemlos bei einer Temperatur oberhalb 35 °C, bevorzugt im Bereich von 20 bis 30 °C.

Ist das Pyridiniumsalz ohne Zusatz von Lösungsmitteln hergestellt worden, kann die Alkalicyanidlösung auch zum Pyridiniumsalz oder zu einer hergestellten wäßrigen Lösung gegeben werden. Die 2-Cyanoisonicotinsäure fällt aus dieser Reaktionslösung als beigefarbener bis weißer Feststoff aus und kann durch einfache mechanische Methoden abgetrennt werden.

Überraschenderweise wird die 2-Cyanoisonicotinsäure auf diese Weise ohne Aufreinigung in einer Reinheit von etwa 98 % und in der theoretischen Ausbeute von 75 bis 80 %, erhalten. Dieses ist besonders deshalb überraschend, weil vergleichbare Umsetzungen von 4-Cyanopyridin zum 2,4-Dicyanopyridin mit aufwendigen Reinigungsoperationen der Zwischenprodukte zur Abtrennung von teerartigen Nebenprodukten verbunden sind, und die Ausbeuten dementsprechend niedrig liegen. Außerdem werden bei vergleichbaren Umsetzungen das Alkylierungsmittel und das Alkalicyanid in hohem Überschuß unter Schutzgasatmosphäre benötigt, während bei der Herstellung der 2-Cyanoisonicotinsäure äquimolare Mengen derselben genügen und die Reaktionsbedingungen milde sind.

So wird z. B. durch die Umsetzung von 4-Cyanopyridin-1-oxid mit Dimethylsulfat das Pyridiniumsalz als dickes, dunkles Öl erhalten, was eine Abtrennung der entstandenen Nebenprodukte vor der weiteren Umsetzung erforderlich macht. Durch die danach erfolgende Reaktion kann das 2,4-Dicyanopyridin nur in einer theoretischen Ausbeute von 54 % erhalten werden.

Die Hydrolyse der in der Literatur bisher nicht beschriebenen 2-Cyanoisonicotinsäure zur 2,4-Pyridindicarbonsäure läßt sich in einer Ausbeute von mindestens 75 % in einfacher Weise durch dem Fachmann bekannte alkalische oder saure Verseifung und anschließende Einstellung des pH-Wertes der Reaktionslösung auf einen Wert im Bereich von 1 bis 4, bevorzugt von 2 bis 3 durchführen. Besonders bevorzugt sind für die saure Verseifung Schwefelsäure und Salzsäure, für die alkalische Verseifung Natron- und Kalilauge.

Die 2,4-Pyridindicarbonsäure wird bei einem pH von 1 bis 4, bevorzugt 2 bis 3 gefällt.

Besonders vorteilhaft ist die saure Verseifung mit Schwefelsäure und anschließende pH-Einstellung mittels Ammoniak, da man auf diese Weise eine völlig aschefreie 2,4-Pyridindicarbonsäure erhält.

Vorteilhaft ist aber auch die Verseifung mit Natron- oder Kalilauge und anschließende pH-Einstellung mit Salzsäure, da man auf diese Weise ein sulfat- und ammoniumhaltiges Abwasser vermeidet.

Obwohl also die Herstellung der 2,4-Pyridindicarbonsäure aus der käuflichen Isonicotinsäure in einer vierstufigen Reaktion verläuft, wird die Pyridindicarbonsäure in vergleichsweise hoher Ausbeute und einer Reinheit von 99 %, erhalten, ohne die Zwischenprodukte oder das Endprodukt aufwendig reinigen, bzw. alle Zwischenprodukte aus den Reaktionslösungen abtrennen zu müssen. Gleichzeitig können durch diese Herstellungsmethode Abwasserprobleme vermieden werden, da die Reaktionspartner in äquimolaren Mengen eingesetzt werden können, bzw. in so geringen Überschüssen eingesetzt werden, daß sie keine Probleme bereiten.

Im Gegensatz zu anderen Wegen sind in diesem Fall keine aufwendigen Sicherheitsmaßnahmen erforderlich, da unter milden Reaktionsbedingungen gearbeitet werden kann und die entsprechenden Zwischen- und Nebenprodukte unbedenklich sind.

### BEISPIELE

### Beispiel 1

### Isonicotinsäure-N-oxid

246 g (2 Mol) Isonicotinsäure werden in 650 ml Eisessig suspendiert und auf 80 °C erhitzt. Hierzu tropft man innerhalb 15 Minuten 102 g (2,1 Mol) Wasserstoffperoxid (70 %). Man läßt 18 Stunden bei 80 bis 90 °C nachreagieren. Danach wird ein Großteil Wasser/Essigsäure abdestilliert, der Rückstand mit 200 ml Wasser versetzt und eingeengt. Der ausfallende Feststoff wird abgesaugt, mit Wasser gewaschen und an der Luft getrocknet.

Ausbeute 267 g (96 %) Isonicotinsäure-N-oxid.

Isonicotinsäure-N-oxid ist ein entzündlicher Feststoff mit einer Zersetzungstemperatur von 290 °C.

### Beispiel 2

### 2-Cyanoisonicotinsäure

27,8 g (0,2 Mol) Isonicotinsäure-N-oxid werden in 80 ml Toluol unter Rühren zum Rückfluß erhitzt. Dabei werden 30,8 g (0,2 Mol) Diethylsulfat innerhalb 30 Minuten zugetropft. Man läßt 3 Stunden bei Rückfluß nachreagieren. Nach dem Abkühlen versetzt man mit 40 ml Wasser und 11,2 g (0,2 Mol) Kaliumhydroxid. Man fügt portionsweise insgesamt 9,8 g (0,2 Mol) Natriumcyanid zu, wobei die Innentemperatur durch Kühlung bei max. 30 °C gehalten wird. Man läßt 2 Stunden nachreagieren. Anschließend wird mit Salzsäure auf ca. pH 2 angesäuert. Die ausgefallene 2-Cyanoisonicotinsäure wird abgesaugt, mit Wasser gewaschen und getrocknet.

Ausbeute 22,8 g (77 %).

¹H-NMR (DMSO-d₆) : δ = 8,10 (d, J = 5,1 Hz, 5-H); 8,37 (s, 3-H); 8,95 (d, J = 5,1 Hz, 6-H); 14,1 (br s, COOH).

### Beispiel 3

### 2-Cyanoisonicotinsäure

41,7 g Isonicotinsäure-N-oxid (hergestellt z. B. nach Ghigi, Ber. 75 (1942), Seiten 1318, bzw. 1322) und 42 ml Diethylsulfat werden 2¹/₂ Stunden bei 105 °C gerührt, nach dem Abkühlen mit 80 ml Wasser versetzt und die entstandene Lösung mit 20,4 g 25 %iger Ammoniaklösung versetzt. Man tropft die ammoniakalische Lösung zu einer Lösung von 15,7 g Natriumcyanid in 65 ml Wasser und läßt eine halbe Stunde nachreagieren. Danach wird mit Schwefelsäure ein pH von 6 bis 7 eingestellt. Der Niederschlag wird abgesaugt und mit 2 x 20 ml H₂O gewaschen. Trocknen bei 55 °C im Vakuum.
Ausbeute 22,2 g (50 %) 2-Cyanoisonicotinsäure.

¹H-NMR (DMSO-d₆): = 8,20 (d, I = 5 Hz, 1H, 5-H); 8,42 (s, 1H, 3-H); 9,02 (d, I = 5 Hz, 1H, 6-H); 14,0 (br, 1H, COOH).

### Beispiel 4

### Pyridin-2,4-dicarbonsäure

5 g (0,034 Mol) 2-Cyanoisonicotinsäure werden mit 4,6 g (0,08 Mol) Kaliumhydroxid in 20 ml Wasser 5 Stunden zum Rückfluß erhitzt. Nach dem Abkühlen versetzt man mit wenig Wasser und säuert mit konz. Salzsäure auf ca. pH 2 an. Die ausgefallene Pyridin-2,4-dicarbonsäure wird abgesaugt, mit Wasser gewaschen und getrocknet.

Ausbeute 4,4 g (78 %).

## Patentansprüche

1. Verfahren zur Herstellung von 2,4-Pyridindicarbonsäure, **dadurch gekennzeichnet**, daß
a) Isonicotinsäure zum Isonicotinsäure-N-oxid oxidiert,
b) Isonicotinsäure-N-oxid in Gegenwart eines Alkylierungsmittels zu einem N-Alkoxy-isonicotinsäuresalz umgesetzt,
c) das Salz durch ein Alkalicyanid in wäßriger Lösung zur 2-Cyanoisonicotinsäure umgesetzt und
d) aus diesem Produkt durch Hydrolyse und anschließende pH-Einstellung auf eine pH-Wert im Bereich von 1 bis 4 2,4-Pyridindicarbonsäure in hochreiner Form gewonnen wird.

2. Verfahren gemäß Anspruch 1, **dadurch gekennzeichnet**, daß Isonicotinsäure in Gegenwart von Wasserstoffperoxid in Essigsäure zum Isonicotinsäure-N-oxid oder in Peressigsäure oxidiert wird.

3. Verfahren gemäß Anspruch 1, **dadurch gekennzeichnet**, daß Isonicotinsäure-N-oxid mittels einem Dialkylsulfat, bevorzugt Dimethyl- oder Diethylsulfat bei 90 bis 105 °C zu einem N-Alkoxy-isonicotinsäuresalz umgesetzt wird.

4. Verfahren gemäß Anspruch 1, **dadurch gekennzeichnet**, daß das gebildete N-Alkoxy-isonicotinsäuresalz in wäßriger Lösung durch ein Natrium- oder Kaliumcyanid zur 2-Cyanoisonicotinsäure umgesetzt wird.

5. Verfahren gemäß Anspruch 1, **dadurch gekennzeichnet**, daß zur sauren Hydrolyse der 2-Cyanoisonicotinsäure Schwefelsäure oder Salzsäure verwendet und anschließend mit Ammoniaklösung ein pH im Bereich von 1 bis 4 eingestellt wird.

6. Verfahren nach Anspruch 1, **dadurch gekennzeichnet**, daß zur alkalischen Hydrolyse der 2-Cyanoisonicotinsäure Natron- oder Kalilauge verwendet wird und anschließend mit Säure ein pH im Bereich von 1 bis 4 eingestellt wird.

7. 2-Cyanoisonicotinsäure

8. Verwendung der 2-Cyanoisonicotinsäure als Zwischenprodukt für die Herstellung von Pyridin-2,4-dicarbonsäure.

## Claims

1. A process for the preparation of 2,4-pyridinedicarboxylic acid, **characterized in that**
a) isonicotinic acid is oxidized to form isonicotinic acid-N-oxide,
b) isonicotinic acid-N-oxide is reacted in the presence of an alkylating agent to form an N-alkoxy-isonicotinic acid salt,
c) the salt is reacted by an alkali cyanide in an aqueous medium to form 2-cyanoisonicotinic acid, and
d) 2,4-pyridinedicarboxylic acid is obtained in an extremely pure form from this product by hydrolysis and by subsequently setting the pH to a value in the range of from 1 to 4.

2. A process according to Claim 1, **characterized in that** isonicotinic acid in the presence of hydrogen peroxide is oxidized in acetic acid to form isonicotinic acid-N-oxide or in peracetic acid.

3. A process according to Claim 1, **characterized in that** isonicotinic acid-N-oxide is reacted by means of a dialkyl sulphate, preferably dimethyl or diethyl sulphate, at from 90 to 105°C to form an N-alkoxy-isonicotinic acid salt.

4. A process according to Claim 1, **characterized in that** the N-alkoxy isonicotinic acid salt formed is reacted in an aqueous solution [with] a sodium or potassium cyanide to form 2-cyanoisonicotinic acid.

5. A process according to Claim 1, **characterized in that** sulphuric acid or hydrochloric acid is used for the acid hydrolysis of the 2-cyanoisonicotinic acid, and a pH in the range of from 1 to 4 is subsequently set with ammonia solution.

6. A process according to Claim 1, **characterized in that** caustic potash solution or caustic soda solution is used for the alkaline hydrolysis of the 2-cyanoisonicotinic acid, and a pH in the range of from 1 to 4 is subsequently set with acid.

7. 2-cyanoisonicotinic acid

8. Use of 2-cyanoisonicotinic acid as an intermediate product for the preparation of pyridine-2,4-dicarboxylic acid.

## Revendications

1. Procédé de préparation de l'acide 2,4-pyridinedicarboxylique, caractérisé en ce que :
a) l'acide isonicotinique est oxydé en N-oxyde de l'acide nicotinique;
b) le N-oxyde de l'acide nicotinique est transformé, en présence d'un agent alcoylant, en un sel d'acide N-alcoxyisonicotinique;
c) le sel est transformé par un cyanure alcalin en solution aqueuse en acide 2-cyanoisonicotinique, et
d) à partir de ce produit, on obtient l'acide 2,4-pyridinedicarboxylique sous forme très pure, par hydrolyse et ensuite ajustement du pH à une valeur dans l'intervalle de 1 à 4.

2. Procédé suivant la revendication 1, caractérisé en ce que l'acide isonicotinique est oxydé en N-oxyde de l'acide isonicotinique en présence de peroxyde d'hydrogène dans de l'acide acétique ou de l'acide peracétique.

3. Procédé suivant la revendication 1, caractérisé en ce que le N-oxyde de l'acide isonicotinique est transformé en un sel d'acide N-alcoxyisonicotinique au moyen d'un dialcoylsulfate, de préférence, le diméthyl- ou le diéthylsulfate, à 90 à 105°C.

4. Procédé suivant la revendication 1, caractérisé en ce que le sel d'acide N-alcoxyisonicotinique formé est transformé en acide 2-cyanoisonicotinique, en solution aqueuse, par un cyanure de sodium ou de potassium.

5. Procédé suivant la revendication 1, caractérisé en ce que l'on utilise de l'acide sulfurique ou de l'acide chlorhydrique pour l'hydrolyse acide de l'acide 2-cyanoisonicotinique et ensuite, que l'on ajuste le pH dans l'intervalle de 1 à 4 avec une solution d'ammoniaque.

6. Procédé suivant la revendication 1, caractérisé en ce que l'on utilise de la lessive de soude ou de potasse pour l'hydrolyse alcaline de l'acide 2-cyanoisonicotinique et ensuite, que l'on ajuste le pH dans l'intervalle de 1 à 4 avec un acide.

7. Acide 2-cyanoisonicotinique.

8. Utilisation de l'acide 2-cyanoisonicotinique comme produit intermédiaire pour la préparation de l'acide 2,4-pyridinedicarboxylique.
